# FASCICULE DE BREVET EUROPEEN

(11) **EP 4 149 579 B1**
(45) Date de publication et mention de la délivrance du brevet: **02.10.2024**
(21) Numéro de dépôt: 21729214.3
(22) Date de dépôt: 12.05.2021
(51) Int. Cl.: A61L 27/34, A61L 27/36

(54) **PROCÉDÉ DE FABRICATION D'UN DISPOSITIF MÉDICAL EN TROIS DIMENSIONS ET DISPOSITIF MÉDICAL OBTENU**
VERFAHREN ZUR HERSTELLUNG EINER DREIDIMENSIONALEN MEDIZINISCHEN VORRICHTUNG UND RESULTIERENDE MEDIZINISCHE VORRICHTUNG
METHOD FOR MANUFACTURING A THREE-DIMENSIONAL MEDICAL DEVICE AND RESULTING MEDICAL DEVICE

(30) Priorité: 12.05.2020 FR 2004641
(43) Date de publication de la demande: 22.03.2023
(73) Titulaire: PH Tech, 33550 Le Tourne (FR)
(72) Inventeur: PERES, Anthony, 17139 DOMPIERRE SUR MER (FR); HOFMANSKI, Guillaume, 61250 MIEUXCE (FR)
(74) Mandataire: Aquinov
(86) Numéro de dépôt international: PCT/EP2021/062604
(87) Numéro de publication internationale: WO 2021/228929

(56) Documents cités:
- US-A1- 2012 040 013
- US-A1- 2018 353 654

## Description

### Domaine technique

L'invention concerne un procédé d'obtention d'un dispositif médical en trois dimensions à partir d'une matrice biologique acellulaire, ainsi que le dispositif médical obtenu et son utilisation en particulier comme implant.

### Etat de l'art

Beaucoup d'applications chirurgicales requièrent le renforcement des tissus mous et/ou le remplacement de sections anatomiques perdues comme par exemple les reconstructions mammaires. Ces interventions nécessitent l'ajout de dispositifs médicaux conjuguant résistance mécanique, souplesse et compatibilité biologique.

A cet effet, les matrices biologiques sont de plus en plus utilisées pour la fabrication de dispositifs médicaux du fait de leur compatibilité biologique. En effet, dans la reconstruction du sein après une mastectomie ou dans le cadre d'une autre opération chirurgicale de reconstitution esthétique du sein, de nombreux implants comprennent une matrice dermique acellulaire qui couvre l'implant/la prothèse et qui est reliée au muscle du grand pectoral, évitant ainsi d'étirer préalablement la peau et les muscles avant la mise en place de l'implant. En outre, une telle matrice aide au soutien des tissus et à leur régénération.

US 2012/040013 A1 décrit un dispositif médical en 3 dimensions utilisable pour la reconstruction mammaire obtenu en mélangeant des particules d'une matrice biologique acellulaire avec une solution d'un polymère dissous dans un solvant puis en mettant en contact ledit mélange avec un milieu aqueux, permettant ainsi que le solvant diffuse pour former un échafaudage tissulaire.

Toutefois, pour les indications chirurgicales nécessitant une forme particulière en trois dimensions l'utilisation d'une matrice biologique acellulaire n'est possible qu'en présence d'un support permettant le maintien en trois dimensions de la matrice biologique. De plus, les techniques actuelles sont complexes et nécessitent soit de perforer la matrice biologique acellulaire pour lui permettre d'épouser une forme en trois dimensions, soit de découper des éléments de la matrice pour les assembler par suture, collage, etc.

Il existe par conséquent un besoin pour des implants biologiques comprenant une matrice biologique acellulaire qui ne nécessitent pas la présence d'un support permettant le maintien en trois dimensions de la matrice biologique ni la mise en oeuvre de techniques impliquant la perforation ou le découpage, la suture ou le collage.

L'objectif de l'invention est de répondre à ce besoin.

### Résumé de l'invention

A cet effet l'invention a pour objet un procédé de fabrication d'un dispositif médical en trois dimensions à partir d'une matrice biologique acellulaire caractérisé en ce qu'il comprend au moins la succession des étapes suivantes :
a/ mouiller une matrice biologique acellulaire avec un liquide, et
b/ conformer la matrice biologique acellulaire mouillée de façon à ce qu'elle prenne une forme en trois dimensions.

Avantageusement, l'étape de mouillage rend la matrice biologique acellulaire souple et malléable, permettant ensuite de la conformer en trois dimensions. Ainsi le procédé permet d'obtenir facilement, rapidement et à coût réduit, un dispositif médical en trois dimensions, utilisable comme implant, sans nécessité de support permettant le maintien en trois dimensions de la matrice biologique, ni de perforation de la matrice biologique.

L'invention a par conséquent également pour objet un dispositif médical en trois dimensions (non plan) comprenant au moins une matrice biologique acellulaire conformée en trois dimensions, ainsi que son utilisation comme implant.

D'autres caractéristiques et avantages ressortiront de la description en détail de l'invention qui va suivre.

### Figures

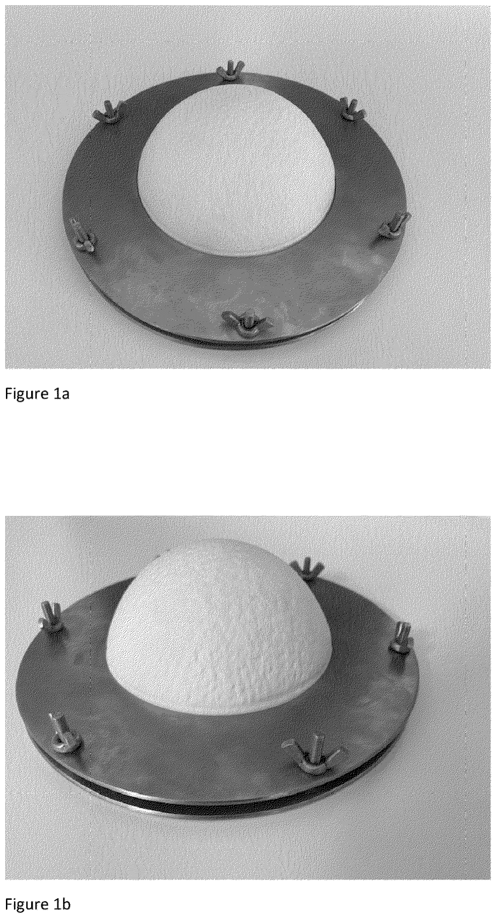

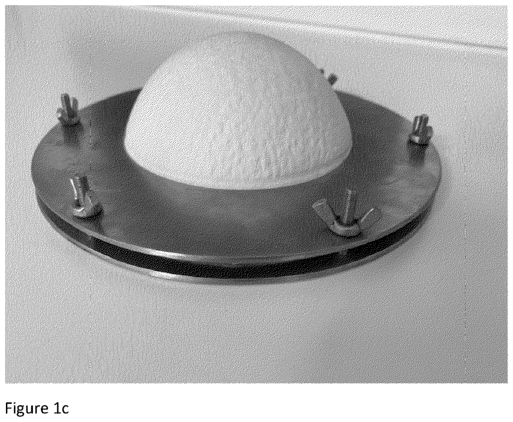

Les figures 1a, 1b et 1c représentent différentes vues d'un exemple de matrice dermique acellulaire déshydratée disposée sur une forme et maintenue sur ladite forme par un disque ou une contre forme, tel que décrit à l'exemple 12.

### Description détaillée de l'invention

### Définitions

Par matrice biologique « acellulaire » au sens de l'invention on entend une matrice biologique dans laquelle les éléments cellulaires ont été éliminés par un procédé de décellularisation dans l'objectif de détruire et/ou enlever les cellules et leurs composants de la matrice extracellulaire de la matrice biologique tout en maintenant sa structure et ses propriétés. En effet, pour qu'une matrice biologique puisse être implantée chez un receveur, elle doit être décellularisée de façon à diminuer son immunogénicité.

Par « Allograft » au sens de l'invention on entend une matrice biologique, un greffon, provenant d'un donneur faisant partie de la même espèce biologique que le receveur.

Par « conformer » ou « conformage » au sens de l'invention on entend l'obtention d'une forme semblable à celle d'un autre objet considéré comme modèle, comme point de référence.

Par « Implant » au sens de l'invention on entend un dispositif médical utilisé en chirurgie.

Par « matrice biologique » au sens de l'invention on entend un biomatériau dérivé de l'espèce humaine ou animale.

Par « P4HB » au sens de l'invention on entend un PHA particulier qui signifie Poly-4-hydroxybutyrate. Il s'agit d'un homopolymère d'unité 4-hydroxybutyrate.

« Peel test » est utilisé ici en référence à un test permettant de déterminer la force d'adhésion entre 2 matériaux. Chaque matériau est placé dans des mâchoires pneumatiques à une pression donnée puis séparé à vitesse constante comme précisé dans les exemples.

Par « PHAs » au sens de l'invention on entend des polyhydroxyalcanoates qui sont des polyesters biodégradables.

Par « trois dimensions » on entend tout objet, en particulier tout dispositif médical, notamment tout implant, non plan.

Par « solution » au sens de l'invention on entend un mélange homogène résultant de la dissolution d'un ou plusieurs soluté(s) dans un solvant.

Par « Suture Rétention Force » (« force de rétention de suture ») ou «Suture Rétention Strength » (« Tension de rétention de suture ») au sens de l'invention on entend un test permettant de déterminer la force (N) nécessaire pour tirer une suture hors d'un spécimen.

Par « Uniaxial tensile Strength » (« résistance uniaxiale à la traction ») au sens de l'invention on entend un test permettant de déterminer la tension avant rupture du spécimen testé. Les propriétés mesurées sont Ultimate Tensile Strength (résistance maximale à la traction), la force de rupture et l'élongation à la rupture.

Par « Viscosité » au sens de l'invention on entend une propriété de résistance à l'écoulement d'un fluide pour un écoulement sans turbulence.

Par « Xenograft » au sens de l'invention on entend une matrice biologique, un greffon, provenant d'un donneur faisant partie d'une espèce biologique différente de celle du receveur.

### Procédé de fabrication d'un dispositif médical en trois dimensions

Selon un premier aspect, l'invention vise un procédé de fabrication d'un dispositif médical en trois dimensions à partir d'une matrice biologique acellulaire.

Les matrices biologiques acellulaires regroupent une large classe de biomatériaux extraits de greffons d'origines diverses.

Préférentiellement la matrice biologique dans le dispositif médical selon l'invention, est d'origine humaine ou animale non humaine (Allograft ou Xenograft).

Selon un mode de réalisation particulièrement adapté il s'agit d'une matrice biologique choisie parmi les matrices biologiques d'origine animale non humaine choisie parmi les matrices biologiques d'origine porcine, bovine, équine, caprine, de poissons et leurs mélanges.

La matrice biologique selon l'invention peut être choisie parmi toutes les matrices biologiques animales et/ou humaines, préférentiellement parmi l'une des matrices biologiques suivantes : derme, sous-muqueuse intestinale, aorte, vessie, membrane amniotique, péritoine, péricarde, dure-mère, tendons, cartilage et leurs mélanges.

La matrice biologique du dispositif médical en trois dimensions selon l'invention est acellulaire. Il existe de nombreux procédés connus permettant d'obtenir une matrice biologique acellulaire. Les procédés utilisés peuvent être enzymatiques et/ou à base de solutions chimiques et/ou s'appuyant sur des procédés mécaniques. Le procédé utilisé doit être un procédé permettant d'obtenir une matrice biologique acellulaire apte à être utilisée en chirurgie en particulier pour la reconstruction des tissus mous.

La matrice biologique acellulaire selon l'invention est préférentiellement une matrice biologique acellulaire qui présente au moins l'une des caractéristiques suivantes, encore plus préférentiellement toutes :
- « Uniaxial Tensile Strength » supérieur ou égal à 5N/mm2 pour un spécimen de 5 mm x 50 mm avec une épaisseur prise en compte dans le calcul. Chaque extrémité est attachée dans des mords pneumatiques dans le sens de la longueur à 1 cm de chaque bord. La vitesse d'écartement des bords est de 30 mm/min. « Uniaxial Tensile Strenght » est reporté en divisant la force maximale (N) / (5 (mm) × épaisseur (mm)).
- « Suture Rétention Force » supérieure ou égale à 5N pour un échantillon de 1cm x 1cm en utilisant une suture appropriée de (type 4-0 polypropylène) enfilée à 3 mm du bord de l'échantillon en son centre, le côté opposé étant clampé dans un mord pneumatique à 5 mm approximativement. Les deux extrémités du fil de suture sont clampées dans le mord inférieur. La vitesse de séparation des mords est de 20mm/min. La force (N) maximum est retenue.

Préférentiellement, la matrice biologique utilisée présente les spécifications définies dans les standards applicables (« USP official monographs » dernière version, ASTM) selon le type de matrice biologique utilisée.

La matrice biologique acellulaire utilisée pour la mise en oeuvre du procédé selon l'invention peut être de forme variée. Préférentiellement elle :
- est sèche, de façon préférée elle présente un taux d'humidité résiduel compris entre 10% et 18%, et/ou
- présente un aspect de surface améliorant l'adhérence d'au moins un polymère, en particulier l'adhérence d'au moins une solution de polymère(s).

La matrice biologique acellulaire utilisée pour la mise en oeuvre du procédé selon l'invention peut avoir différentes formes (rondes, carrés, circulaires, irrégulières ...), plane ou en relief, avec des canaux, avec l'une des surfaces texturées, être d'épaisseur régulière ou irrégulière.

Le procédé selon l'invention de fabrication d'un dispositif médical en trois dimensions à partir d'une matrice biologique acellulaire comprend au moins la succession des étapes suivantes : a/ mouiller une matrice biologique acellulaire avec un liquide, préférentiellement de l'eau, et b/ conformer la matrice biologique acellulaire mouillée de façon à ce qu'elle prenne une forme en trois dimensions.

L'étape a/ qui consiste à mouiller la matrice biologique acellulaire a pour objectif de rendre ladite matrice biologique acellulaire souple et malléable de façon à permettre ensuite son conformage. Préférentiellement, l'étape a/ consistant à mouiller la matrice biologique acellulaire est réalisée en trempant la matrice biologique acellulaire dans un volume d'eau permettant son immersion. De façon préférée le trempage est réalisé pendant une durée 30 secondes à 20 minutes.

L'eau peut être de différentes qualités (apyrogène, eau purifiée, etc....). Selon un mode de réalisation particulièrement adapté l'étape a/ est réalisée avec de l'eau apyrogène.

De façon préférée, lors de l'étape a/ de mouillage la matrice biologique acellulaire devient grise, c'est-à-dire vitreuse, plus particulièrement elle passe de la couleur blanche à la couleur grise (vitreux).

Après mouillage la matrice biologique acellulaire peut être conformée en trois dimensions. Préférentiellement l'étape b/ de conformage est réalisée par formage par le vide (« Vacuum forming ») et/ou moulage humide et/ou pressage.

Dans le cas du conformage par formage sous vide, la matrice est placée sur la forme ou le moule désiré. L'ensemble est alors placé dans un système permettant le maintien de la matrice sur le moule, la forme pendant le temps nécessaire au traitement par formage sous vide. Ledit système peut être composé d'un sac sous vide type alimentaire d'épaisseurs variables (80 à 200 microns) et/ou d'une contre-forme pleine ou ajourée ou présentant simplement des sections similaires à la forme (embase) maintenue sur la matrice formée par tous moyens pendant et après traitement. L'ensemble : « forme + matrice formée » et système de maintien sont ensuite placés dans un système de séchage (lyophilisateur, étuve...).

Dans un mode de réalisation préférée la matrice biologique mouillée est placée sur une forme (ou dans un moule) perforée. L'ensemble est alors placé dans un sac sous vide pour traitement dans une machine sous vide type Henkelman. Le vide est alors réalisé sur un cycle de 10 à 60 secondes entre -1 bar et -2 bars, préférentiellement - 1 bar. Le couple temps de traitement / taux de vide peut être apprécié par l'homme de l'art en fonction du résultat souhaité. La matrice est alors conformée et maintenue dans cet état par le sac sous vide.

Dans un autre mode de réalisation, les matrices biologiques peuvent être pressées dans une presse hydraulique sur une durée de 30 à 60 s, entre 6 bars et 200 bars, notamment entre 50 et 200 bars et à une température entre 50°C et 190°C, notamment entre 50°C et 100°C. Les matrices biologiques planes (et par conséquent les dispositifs médicaux selon l'invention plans) sont traitées dans une presse hydraulique entre 2 plateaux. Les matrices biologiques (et par conséquent les dispositifs médicaux selon l'invention) en relief (type implants biologiques traités par ladite méthode en forme d'implants mammaires de formes anatomiques ou hémisphériques) peuvent être pressés dans des moules disposant des empreintes souhaitées (type « molding machine for fabric cup » (presse à bonnets soutien-gorge). Dans ce cas précis la matrice humide est placée sur une forme puis une contre-forme est positionnée au-dessus de sorte que la matrice est prise « en sandwich » entre les 2 formes. Un couple température/pression dans les grandeurs précédentes est appliqué durant 30 à 60 s. L'ensemble est maintenu après pressage pour permettre le séchage soit par étuve à air chaud soit préférentiellement par lyophilisation de façon à obtenir une humidité relative voulue. L'ensemble matrice forme et contre-forme peut également être lyophiliser avant l'application du couple pression/température de façon à permettre la prise de forme. Ledit couple est alors appliqué pour marquer l'ensemble et permettre le maintien de la forme 3D (notamment mammaire) après hydratation.

Préférentiellement une méthode de séchage qui ne dénature pas le collagène par des températures trop élevées est privilégiée. Le sac sous vide contenant la forme et la matrice conformée est alors placé dans un lyophilisateur pour séchage. Préférentiellement ledit sac peut être placé dans un congélateur à -40°C pour congélation. Après congélation la matrice conformée et la forme sont placées dans un lyophilisateur pour séchage. Les cycles de lyophilisation des matrices biologiques sont connus de l'homme de l'art. La matrice peut également être sortie du sac et placée avec la forme dans un lyophilisateur pour séchage, le cycle de lyophilisation comprend alors une étape de congélation. Dans le cas du pressage, la matrice est placée dans une presse type Short Nose Evolution de chez Maugin. La machine est préalablement équipée d'un moule sur la partie inférieure et d'une contre-forme sur le piston. L'opération consiste alors à venir presser la matrice biologique entre les deux éléments durant un temps défini (entre 30s et 120s) et une pression choisie (entre 6 et 200 bars, notamment entre 50 bars et 200 bars). Une température peut également être régulée (entre 50°C et 190°C, préférentiellement entre 50°C et 150°C, notamment entre 50°C et 100°C). La matrice ainsi formée est alors séchée sur une forme adaptée.

Dans le cas du moulage humide, la matrice est placée sur une forme puis façonnée sur la forme à l'aide d'un repoussoir. La pièce est maintenue sur la forme par fixations mécaniques (agrafage...). Selon une variante de l'invention, le procédé de fabrication d'un dispositif médical selon l'invention comprend également :
- avant ou après l'étape a/ ou avant ou après l'étape b/, une étape de préparation de la matrice biologique acellulaire de façon à permettre l'adhérence sur ladite matrice biologique acellulaire d'une solution comprenant au moins un polymère, et
- avant ou après a/ ou avant ou après l'étape b/, une étape d'enduction partielle ou totale de la matrice biologique acellulaire avec une solution comprenant au moins un polymère. L'étape d'enduction n'intervient qu'après l'étape de préparation de la matrice biologique acellulaire de façon à permettre l'adhérence sur ladite matrice biologique acellulaire de la solution comprenant au moins un polymère utilisé pour l'enduction.

Selon un premier mode de réalisation de cette variante, le procédé de fabrication d'un dispositif médical selon l'invention comprend au moins la succession des étapes suivantes :
- 1/ préparer la matrice biologique acellulaire de façon à permettre l'adhérence sur ladite matrice biologique acellulaire d'une solution comprenant au moins un polymère,
- 2/ enduire partiellement ou totalement la matrice biologique acellulaire avec une solution comprenant au moins un polymère,
- 3/ mouiller la matrice biologique acellulaire avec un liquide,
- 4/ conformer la matrice biologique acellulaire mouillée et enduite de façon à ce qu'elle prenne une forme en trois dimensions.

Selon un deuxième mode de réalisation de cette variante, le procédé de fabrication d'un dispositif médical selon l'invention comprend au moins la succession des étapes suivantes :
- 1/ préparer la matrice biologique acellulaire de façon à permettre l'adhérence sur ladite matrice biologique acellulaire d'une solution comprenant au moins un polymère,
- 2/ mouiller la matrice biologique acellulaire avec un liquide,
- 3/ enduire partiellement ou totalement la matrice biologique acellulaire avec une solution comprenant au moins un polymère,
- 4/ conformer la matrice biologique acellulaire mouillée et enduite de façon à ce qu'elle prenne une forme en trois dimensions.

Selon un troisième mode de réalisation de cette variante, le procédé de fabrication d'un dispositif médical selon l'invention comprend au moins la succession des étapes suivantes :
- 1/ préparer la matrice biologique acellulaire de façon à permettre l'adhérence sur ladite matrice biologique acellulaire d'une solution comprenant au moins un polymère,
- 2/ mouiller la matrice biologique acellulaire avec un liquide,
- 3/ conformer la matrice biologique acellulaire mouillée de façon à ce qu'elle prenne une forme en trois dimensions.
- 4/ enduire partiellement ou totalement la matrice biologique acellulaire avec une solution comprenant au moins un polymère.

Selon un quatrième mode de réalisation de cette variante, le procédé de fabrication d'un dispositif médical selon l'invention comprend au moins la succession des étapes suivantes :
- 1/ mouiller la matrice biologique acellulaire avec un liquide,
- 2/ préparer la matrice biologique acellulaire de façon à permettre l'adhérence sur ladite matrice biologique acellulaire d'une solution comprenant au moins un polymère,
- 3/ enduire partiellement ou totalement la matrice biologique acellulaire avec une solution comprenant au moins un polymère.
- 4/ conformer la matrice biologique acellulaire mouillée et enduite de façon à ce qu'elle prenne une forme en trois dimensions.

Selon un cinquième mode de réalisation de cette variante, le procédé de fabrication d'un dispositif médical selon l'invention comprend au moins la succession des étapes suivantes :
- 1/ mouiller la matrice biologique acellulaire avec un liquide,
- 2/ préparer la matrice biologique acellulaire de façon à permettre l'adhérence sur ladite matrice biologique acellulaire d'une solution comprenant au moins un polymère,
- 3/ conformer la matrice biologique acellulaire mouillée de façon à ce qu'elle prenne une forme en trois dimensions.
- 4/ enduire partiellement ou totalement la matrice biologique acellulaire avec une solution comprenant au moins un polymère.

Selon un sixième mode de réalisation de cette variante, le procédé de fabrication d'un dispositif médical en trois dimensions selon l'invention comprend au moins la succession des étapes suivantes :
- 1/ mouiller la matrice biologique acellulaire avec un liquide,
- 2/ conformer la matrice biologique acellulaire mouillée de façon à ce qu'elle prenne une forme en trois dimensions.
- 3/ préparer la matrice biologique acellulaire de façon à permettre l'adhérence sur ladite matrice biologique acellulaire d'une solution comprenant au moins un polymère,
- 4/ enduire partiellement ou totalement la matrice biologique acellulaire avec une solution comprenant au moins un polymère.

Préférentiellement l'étape de préparation de la matrice biologique cellulaire consiste en un traitement de surface chimique et/ou mécanique et/ou électrochimique et/ou physique. Il peut s'agir par exemple d'un traitement par abrasion et/ou fraisage et/ou microtexturation et/ou laser et/ou UV. La matrice biologique acellulaire avant enduction doit en effet être préparée de façon à permettre l'adhérence d'une solution de polymère(s).

Dans un mode de réalisation préférée, la matrice biologique acellulaire avant enduction et/ou après enduction, doit être sèche ou séchée de façon à présenter un taux d'humidité résiduel de l'ordre de 10% à 18%. Le taux d'humidité résiduel est préférentiellement mesuré à l'aide d'un dessiccateur type Halogen Moisture Analyzer de marque Mettler Toledo.

Une technique de séchage utilisée est préférentiellement celle du Loss on Drying décrite dans l'USP 41 (scaffold bovine dermis):
- 1- une coupelle vide en aluminium est positionnée dans l'appareil et la tare est réalisée,
- 2- la coupelle est remplie avec un échantillon de 1,0 g +/- 0,2 g coupé en morceaux de 4 mm²,
- 3- le programme de chauffe à 130°C est lancé
- 4- lorsque le poids ne varie plus sur un temps donné, le résultat est affiché en %.

Une autre méthode de séchage par étuve conventionnelle peut également être utilisée. Dans ce cadre une coupelle en aluminium préalablement pesée à vide est remplie avec un échantillon de 5,0 g +/- 0,2 g coupé en morceaux 4 mm². L'ensemble est placé à 100°C pendant 16h. L'ensemble est alors pesé et le « loss on drying » (perte au séchage) est calculé :
- matière sèche % = [(poids de l'extrait sec + coupelle (g) - poids de la coupelle (g))/g de l'échantillon] x 100
- humidité % = 100 - matière sèche %.

La matrice biologique préparée est ensuite utilisée pour l'enduction d'une solution de polymères.

Le ou les polymères utilisé(s) pour enduire la matrice biologique acellulaire dans le procédé selon l'invention sont préférentiellement choisis parmi les polymères suivants : poly(glycolides), poly(lactide-co-glycolides); poly(lactic acid), poly(glycolic acid), poly(lactic acid-co-glycolic acids), polycaprolactones, poly(orthoesters), polyanhydrides, poly(phosphazenes), polyhydroxyalkanoates (incluant notamment P4HB et poly-3-hydroxybutyrate-co-3- hydroxy valerate (PHBV)), polyesters, poly(lactide-co-caprolactones), polycarbonates, tyrosine polycarbonates, polyamides, polyesteramides, poly(dioxanones), poly(alkylene alkylates), polyethers, polyvinyl pyrrolidones ou PVP, polyurethanes, polyetheresters, polyacetals, polycyanoacrylates, poly(oxyethylene)/poly(oxypropylene) copolymers, polyacetals, polyketals, polyphosphates, polyphosphoesters, polyalkylene oxalates, polyalkylene succinates, poly(maleic acids), chitin, chitosan, et leurs mélanges.

Préférentiellement la solution comprenant au moins un polymère a été préalablement obtenue par solubilisation du ou des polymère(s) sec(s) dans au moins un solvant, préférentiellement au moins un solvant polaire.

Le ou les polymères doivent en effet préférentiellement être convertis en une solution propre à permettre l'enduction en utilisant le solvant adéquat.

Lorsque le polymère est un PHA et en particulier le P4HB et/ou un de ses copolymères, le solvant est préférentiellement choisi parmi les solvants polaires suivants : dichlorométhane, chloroforme, tetrahydrofurane, dioxane, acétone et leurs mélanges.

Dans un mode de réalisation préféré, le P4HB est solubilisé dans une solution d'acétone, préférentiellement selon un ratio 5% à 25% (w/w). Les quantités respectives sont mises en présence, l'ensemble est chauffé et maintenu à une température inférieure au point d'ébullition de l'acétone (approximativement 56°C) jusqu'à dissolution complète du P4HB et obtention de la viscosité désirée au moins 10%, plus préférablement 15% et encore plus préférablement 20% (w/w). Préférentiellement, après solubilisation du ou des polymère(s) dans un solvant, la solution de polymère(s) est dégazée et/ou débullée pour purger le mélange de bulles d'air. De façon préférée la solution est placée sous vide (minimum -1 bar) pendant le temps nécessaire à un dégazage / débullage complet.

L'étape d'enduction de la matrice biologique acellulaire par une solution de polymère(s) est préférentiellement réalisée à une température inférieure ou égale à la température de dénaturation du collagène. De façon particulièrement adaptée, l'enduction est réalisée à une température comprise entre 10 et 60°C, préférentiellement entre 10 et 50°C, encore plus préférentiellement entre 20 et 50°C.

L'enduction peut être réalisée par tout moyen adapté, préférentiellement par solvant casting (enduction par coulage), spray coating (enduction par vaporisation), dip coating (enduction par immersion).

Avantageusement, le procédé selon l'invention permet l'application directe d'une solution de polymère(s) à la concentration désirée sur une matrice biologique acellulaire préalablement préparé et il n'est pas nécessaire de préalablement fabriquer un film ou une feuille de polymère pour ensuite le disposer sur le support afin de permettre l'adhésion des éléments entre eux par chauffage. Lorsqu'elle est réalisée par « solvent casting », l'enduction peut être réalisée avec différentes technologies : knife (couteau), double side (double face), commabar, case knife (couteau), engraved roller (rouleau engravé), 2 relier (2 rouleaux), 3 roller combi (combi 3 rouleaux), micro relier (micro rouleau), 5 roller (5 rouleaux), reverse relier (rouleau reversé), rotary screen (rotation), dipping (immersion), slot Die (fente), curtain coating (enduction rideau), hotmelt slot die (fondoir). La méthode la plus simple repose sur l'utilisation d'un casting knife (couteau de coulée) type Elcometer. La matrice biologique est disposée sur une table. En fonction de l'épaisseur souhaitée et de la surface à traiter, la quantité nécessaire de solution de polymère(s) est disposée sur la matrice biologique acellulaire. Le Gardener Knife (couteau de jardinier) est alors déplacé sur la matrice biologique acellulaire afin d'uniformiser l'enduction sur l'implant. Le Gardener Knife a préalablement été ajusté à une certaine hauteur.

Dans le but d'automatiser l'opération de « coating machine » (machine de revêtement) peut être utilisée. La solution de polymère(s) est pompée à travers un « slot die » (matrice de fente) pour être appliquée sur l'implant biologique plan en mouvement. Dans un mode de réalisation préféré la largeur du slot die est de 600 mm, la vitesse de progression de l'implant est de 1 - 10 m/min. La vitesse de pompage, la vitesse de progression, la largeur du « slot die » et la concentration de la solution peuvent être ajustées afin d'obtenir l'implant avec une enduction d'épaisseur et de largeur souhaitées.

Lorsque l'enduction est réalisée par « spray coating » ou pulvérisation, la solution de polymère(s) est pompée jusqu'à une buse qui projette des gouttelettes sur la surface à traiter. Cette technique est particulièrement avantageuse pour le traitement de matrices biologiques qui présentent des formes 3D (exemple : implant biologique ayant une forme hémisphérique, ovoide, tubulaire, en forme d'implants mammaires anatomiques....).

Lorsque l'enduction est réalisée par « Dip coating » (revêtement par immersion), ou par trempage, la pièce à traiter est trempée dans une matière dissoute, fondue, ramollie ou en poudre fluidisée afin de la recouvrir d'une couche de cette matière. Cette technique est particulièrement avantageuse pour le traitement d'implants biologiques en relief et plan.

Après enduction, le dispositif médical obtenu est composé d'une couche de matrice biologique enduite partiellement ou totalement d'au moins un polymère en solution, et l'ensemble étant ensuite séché puis préférentiellement pressé pour obtenir une épaisseur uniforme.

Dans un mode de réalisation préféré les matrices biologiques acellulaires enduites par la solution de polymère(s) sont placées dans une étuve ou un four ou une enceinte de chauffe afin de permettre l'évaporation complète du solvant. A une température entre 0°C et 50°C, comprise entre 15 et 40°C, préférablement 30°C plus ou moins 5°, de façon à éviter une évaporation trop rapide et une déformation de la matrice biologique.

Selon une variante particulière de l'invention, les matrices biologiques acellulaires enduites ou non par la solution de polymère(s), éventuellement avant ou après séchage, sont pressées dans une presse hydraulique sur une durée de 30 à 60 s, entre 6 bars et 200 bars, notamment entre 50 et 200 bars et à une température entre 50°C et 190°C, notamment entre 50°C et 100°C. Les matrices biologiques planes (et par conséquent les dispositifs médicaux selon l'invention plans) sont traitées dans une presse hydraulique entre 2 plateaux. Les matrices biologiques (et par conséquent les dispositifs médicaux selon l'invention) en relief (type implants biologiques traités par ladite méthode en forme d'implants mammaires de formes anatomiques ou hémisphériques) peuvent être pressés dans des moules disposant des empreintes souhaitées (type « molding machine for fabric cup » (presse à bonnets soutien-gorge). Dans ce cas précis la matrice humide est placée sur une forme puis une contre-forme est positionnée au-dessus de sorte que la matrice est prise « en sandwich » entre les 2 formes. Un couple température/pression dans les grandeurs précédentes est appliqué durant 30 à 60 s. L'ensemble est maintenu après pressage pour permettre le séchage soit par étuve à air chaud soit préférentiellement par lyophilisation de façon à obtenir une humidité relative telle que définit plus avant. L'ensemble matrice forme et contre-forme peut également être lyophiliser avant l'application du couple pression/température de façon à permettre la prise de forme. Ledit couple est alors appliqué pour marquer l'ensemble et permettre le maintien de la forme 3D (notamment mammaire) après hydratation. La mise en forme décrite ci-avant peut être réalisée sur un implant enduit ou non de sorte que la matrice formée seule peut-être enduite après mise en forme selon les procédés décrits dans les sections précédentes.

### Dispositif médical en trois dimensions et utilisations

Selon un deuxième aspect, l'invention vise aussi spécifiquement un dispositif médical en trois dimensions, susceptible d'être obtenu par la mise en oeuvre du procédé selon l'invention, comprenant au moins une matrice biologique acellulaire conformée en trois dimensions.

Avantageusement, le dispositif médical en trois dimensions selon l'invention ne comprend pas de support pour le maintien en trois dimensions de la matrice biologique acellulaire, ladite matrice biologique ayant été conformée en trois dimensions par des procédés de conformage réalisée sur la matrice biologique acellulaire préalablement mouillée (préférentiellement immergée dans l'eau). Préférentiellement, le dispositif médical en trois dimensions selon l'invention comprend au moins une matrice biologique acellulaire recouverte partiellement ou totalement par au moins une couche comprenant au moins un polymère.

Le dispositif médical en trois dimensions selon l'invention peut comprendre une ou plusieurs matrices biologiques acellulaires, une ou plusieurs couches de polymère(s) et éventuellement d'autres constituants. Le dispositif médical en trois dimensions selon l'invention peut comprendre par exemple au moins une matrice biologique acellulaire recouverte partiellement ou totalement par au moins deux couches comprenant au moins un polymère.

Selon un mode de réalisation particulier, le dispositif médical en trois dimensions est constitué exclusivement par une matrice biologique acellulaire recouverte partiellement ou totalement par une couche comprenant au moins un polymère.

Selon un autre mode de réalisation particulier, le dispositif médical en trois dimensions est constitué exclusivement par une matrice biologique acellulaire recouverte partiellement ou totalement par deux couches comprenant au moins un polymère.

La présence du ou des polymères dans le dispositif médical en trois dimensions selon l'invention permet un renfort optimal durant la reconstruction tissulaire supportée par la matrice biologique et cela même en cas d'infection. En effet, la présence d'au moins un polymère avec la matrice biologique acellulaire permet de rendre la matrice biologique résistante aux infections, tout en conservant les qualités de la matrice biologique en termes de résistance mécanique, souplesse et comptabilité biologique. Il peut ainsi être utilisé pour des applications médicales en particulier en chirurgie.

Le polymère présent dans le dispositif médical en trois dimensions selon l'invention peut être tout type de polymère adapté à une utilisation comme dispositif médical et en particulier comme implant, notamment comme implant biologique, en particulier comme implant chirurgical ou implant biologique chirurgical.

Le ou les polymères utilisé(s) présent(s) dans le dispositif médicale en trois dimensions selon l'invention sont préférentiellement choisis parmi les polymères suivants : poly(glycolides), poly(lactide-co-glycolides); poly(lactic acid), poly(glycolic acid), poly(lactic acid-co-glycolic acids), polycaprolactones, poly(orthoesters), polyanhydrides, poly(phosphazenes), polyhydroxyalkanoates (incluant notamment P4HB et poly-3-hydroxybutyrate-co-3- hydroxy valerate (PHBV)), polyesters, poly(lactide-co-caprolactones), polycarbonates, tyrosine polycarbonates, polyamides, polyesteramides, poly(dioxanones), poly(alkylene alkylates), polyethers, polyvinyl pyrrolidones ou PVP, polyurethanes, polyetheresters, polyacetals, polycyanoacrylates, poly(oxyethylene)/poly(oxypropylene) copolymers, polyacetals, polyketals, polyphosphates, polyphosphoesters, polyalkylene oxalates, polyalkylene succinates, poly(maleic acids), chitin, chitosan, et leurs mélanges.

Selon un mode de réalisation particulièrement adapté, le dispositif médical en trois dimensions selon l'invention comprend au moins un PHA et encore plus préférentiellement au moins un PHA choisi parmi le P4HB, les copolymères du P4HB et leurs mélanges. Les PHAs constituent une famille de matériaux produits par de nombreux micro-organismes. On peut citer par exemple le brevet US 6,316,262 de la société Metabolix, Inc. Of Cambridge MA, USA, qui décrit une méthode permettant l'obtention d'un système biologique permettant la production de polymères de polyhydroxyalkanoate contenant du 4-hydroxyacides. Les brevets US 6,245,537, US 6.623.748, US 7,244,442 et US 8,231,889 décrivent également des méthodes d'élaboration de PHAs adaptées à une utilisation médicale et à des dispositifs médicaux selon l'invention.

Préférentiellement le PHA et en particulier le P4HB et/ou ses copolymères présentent un niveau bas d'endotoxines, en particulier ils permettent d'avoir taux inférieur à 20 EU par dispositif médical.

La ou les couches de polymères peu(ven)t comprendre un ou plusieurs canaux qui permettent d'incorporer lors de l'utilisation en chirurgie, des éléments favorisant une reconstruction optimale des tissus dans lequel le dispositif est utilisé comme implant (PRP, cellules souches, antibiotiques, etc). Ces canaux peuvent être circulaires ou non circulaires. Ils ont préférentiellement une surface interne comprise entre 0,007 mm² et 0,8mm². Ils peuvent être obtenus par impression d'une forme dans la matrice biologique par pressage avant enduction de la matrice biologique. La forme étant alors retirée après enduction et séchage.

Le dispositif médical selon l'invention est préférentiellement utilisé comme implant biologique, en particulier comme implant chirurgical ou implant biologique chirurgical. Selon une variante il s'agit d'un implant pour reconstruction mammaire.

En particulier et de façon non limitative les dispositifs médicaux en trois dimensions selon l'invention peuvent être utilisés pour les applications suivantes : réparation, régénération et remplacement des tissus mous et durs, dispositif de cicatrisation, bandage, patch, pansement, pansement pour brûlures, pansement pour ulcère, substitut cutané, hémostatique, dispositif de reconstruction trachéale, dispositif de sauvetage d'organes, substitut dural, patch dural, nerf guide, dispositif de régénération ou de réparation nerveuse, dispositif de réparation de hernie, maille de hernie, bouchon de hernie, dispositif de support temporaire de plaie ou de tissu, échafaudage d'ingénierie tissulaire, dispositif de réparation / régénération de tissu guidé, dispositifs de fixation pour mailles, membrane anti-adhérence, adhérence barrière, membrane de séparation des tissus, membrane de rétention, élingue, dispositif de reconstruction du plancher pelvien, dispositif de suspension urétrale, dispositif de traitement de l'incontinence urinaire, dispositif de réparation de la vessie, dispositif de gonflement ou de remplissage, dispositif de réparation de la coiffe des rotateurs, dispositif de réparation du ménisque, dispositif de régénération du ménisque, membrane de régénération tissulaire guidée pour les tissus parodontaux, dispositif d'anastomose, dispositif ensemencé de cellules, dispositif d'encapsulation cellulaire, dispositif de libération contrôlée, dispositif d'administration de médicaments, dispositif de chirurgie plastique, dispositif de lifting mammaire, dispositif de mastopexie, dispositif de reconstruction mammaire, dispositif d'augmentation mammaire, dispositif de réduction mammaire, appareils de reconstruction mammaire après mastectomie avec ou sans implants mammaires, appareil de rhinoplastie.

L'invention est à présent illustrée par des exemples et des résultats d'essai.

### Exemples et résultats d'essais

### Exemple 1 : mouillage et conformage

Une matrice dermique acellulaire est mouillée comme suit : elle est immergée dans un volume d'eau purifiée de type 2 pendant 15 min.

Elle est ensuite placée sur une forme mammaire anatomique elle-même vissée sur un plateau perforé. La matrice dermique est fixée sur les rebords du plateau par des fixations mécaniques (agrafages, cadre périphérique...). L'ensemble est placé dans un sac sous vide de 80 micron. Le traitement est effectué par une machine type Henkelman Marlin. Le traitement par le vide est effectué à - 1bar.

Après traitement l'ensemble est placé dans un congélateur à -40 °C. Une fois congelé, le contenu est sorti du sac puis placé dans un lyophilisateur pour séchage.

Préférentiellement la matrice est stérilisée.

La matrice biologique acellulaire ainsi conformée en trois dimensions peut être utilisée comme dispositif médical en particulier comme implant notamment en chirurgie.

### Exemple 2 : mouillage et conformage

Une matrice dermique est mouillée comme suit : elle est immergée dans un volume d'eau apyrogène pendant 3 min.

Elle est ensuite placée dans une presse type Short Nose Evolution de chez Maugin. Une pression est exercée à 100 bars pendant 30s et à une température de 100°C. La matrice ainsi traitée est placée dans une étuve pour séchage à 40°C pendant une nuit.

Après séchage les implants ainsi formés peuvent être pressés afin d'uniformiser l'épaisseur dans une presse hydraulique équipée de la forme et contre-forme adaptée. L'espace entre les deux étant réglé de manière à contrôler l'épaisseur souhaitée. Le temps de pressage variant entre 30s et 60s et la pression entre 50 bars et 200 bars.

La matrice biologique acellulaire ainsi conformée en trois dimensions peut être utilisée comme dispositif médical en particulier comme implant notamment en chirurgie.

### Exemple 3 : traitement de la matrice biologique acellulaire

Une matrice dermique acellulaire plane sèche (taux d'humidité résiduel compris entre 10% et 18%) est placée sur une machine-outil à commande numérique. Une fraise carbure diamètre 5 mm est montée sur la machine-outil. Une vitesse de rotation de 20000 à 40000 tours/min est utilisée avec une avance de 2 m/min. La profondeur est variable en fonction de l'épaisseur finale souhaitée. Le surfaçage peut être total ou ne concerner qu'une partie de l'implant de manière à délimiter des formes.

La matrice est ensuite enduite par une solution de polymère(s), préférentiellement selon l'exemple 6.

La matrice ensuite est ensuite mouillée et conformée selon l'un des exemples 1 ou 2.

La matrice est ensuite préférentiellement séchée de façon à présenter un taux d'humidité résiduel compris entre 10% et 18%.

### Exemple 4 : traitement de la matrice biologique acellulaire

Une matrice dermique acellulaire plane sèche (taux d'humidité résiduel compris entre 10% et 18%) est placée sur une machine brosseuse / cardeuse. La surface de l'implant est ainsi traitée par des cardes de diamètre souhaitée (par exemple 105 mm) composées de fils métalliques (de diamètre par exemple 0,2 mm).

La matrice est ensuite enduite par une solution de polymère(s).

La matrice ensuite est ensuite mouillée et conformée selon l'un des exemples 1 ou 2.

La matrice est ensuite préférentiellement séchée de façon à présenter un taux d'humidité résiduel compris entre 10% et 18%.

### Exemple 5 : traitement de la matrice biologique acellulaire

Une matrice dermique acellulaire sèche (taux d'humidité résiduel compris entre 10% et 18%) est traitée avec un champ électrique haute fréquence type Telea Biotech (4-64 MHz) de façon à créer des cavités et/ou perforations de 0,6mm de diamètre.

La matrice est ensuite enduite par une solution de polymère(s).

La matrice ensuite est ensuite mouillée et conformée selon l'un des exemples 1 ou 2.

La matrice est ensuite préférentiellement séchée de façon à présenter un taux d'humidité résiduel compris entre 10% et 18%.

### Exemple 6 : traitement de la matrice biologique acellulaire

Une matrice dermique acellulaire est traitée comme précisé dans l'exemple 3. Une fraise carbure de 2 mm est montée sur la machine-outil afin de dessiner dans la matrice des canaux en forme sinusoïdale sur la longueur de la matrice. Vitesse et avance sont similaires à l'exemple 1. Les canaux sont répétés à intervalle régulier sur la largeur de façon à couvrir une partie ou la totalité de l'implant. La profondeur des canaux est fonction du diamètre final désiré. Dans un autre mode de réalisation une empreinte peut être fabriquée de façon à imprimer par pressage les canaux dans la matrice.

La matrice est ensuite enduite par une solution de polymère(s).

La matrice ensuite est ensuite mouillée et conformée selon l'un des exemples 1 ou 2.

La matrice est ensuite préférentiellement séchée de façon à présenter un taux d'humidité résiduel compris entre 10% et 18%.

### Exemple 7 : exemple de procédé d'enduction par « solvent casting »

Une matrice dermique acellulaire traitée selon l'exemple 3 de dimensions 2 cm × 6 cm soit 12 cm² est disposée sur une table. La quantité souhaitée de P4HB étant de 0,0164 g/cm², une solution P4HB/acétone à 18% (w/w) est préparée. La quantité nécessaire est prélevée et déposée sur l'implant. Le casting knife est alors déplacé par translation sur l'implant afin d'uniformiser l'épaisseur de l'enduction. Dans le but d'automatiser l'opération une « coating machine » peut être utilisée. Une solution acétone/P4HB est préparée afin d'alimenter la machine. La solution est pompée à travers un « slot die » pour être appliquée sur l'implant biologique plan en mouvement. Dans un mode de réalisation préféré la largeur du slot die est de 600 mm, la vitesse de progression de l'implant est de 1-10 m/min.

La matrice ensuite est ensuite mouillée et conformée selon l'un des exemples 1 ou 2.

La matrice est ensuite préférentiellement séchée de façon à présenter un taux d'humidité résiduel compris entre 10% et 18%.

### Exemple 8 : exemple de procédé de dip coating

Une matrice biologique acellulaire traitée selon l'exemple 4 de 4 cm × 6 cm est immergée par une machine (l'implant est disposé sur une traverse verticale) dans un bac contenant une solution acétone/P4HB à la concentration souhaitée. La matrice biologique est alors extraite du bac par un mouvement vertical ascendant à une vitesse de 25mm/min. La densité de P4HB obtenue par l'enduction est alors de 0,02 à 0,04 g/cm². Les paramètres (taille de la pièce à traiter, vitesse de la traverse, concentration de la solution) sont appréhendables par l'homme de l'art afin d'obtenir l'enduction souhaitée in fine.

La matrice ensuite est ensuite mouillée et conformée selon l'un des exemples 1 ou 2.

La matrice est ensuite préférentiellement séchée de façon à présenter un taux d'humidité résiduel compris entre 10% et 18%.

### Exemple 9 : exemple de séchage et pressage et caractéristiques des dispositifs médicaux obtenus

Une matrice dermique traitée selon l'exemple 3 est enduite à approximativement 0,03g/cm² de P4HB et est pressée pendant 30s à 100 bars de pression et 50°C (plateau presse préalablement chauffée). Sur 3 éprouvettes testées, d'une épaisseur moyenne 1,35 mm, l'uniaxial tensile strenght (UTS) max moyen est de 26,50 MPa.

Le test est réalisé sur un banc de mesure Instron modèle 3342/L2345. Le spécimen est découpé avec un emporte-pièce type bone shape type V cutter tel que décrit dans le standard ASTM D-638-5. La pièce ainsi découpée est introduite par chaque extrémité dans les mords pneumatiques du banc (60psi) en laissant une partie centrale de 2,5 cm. Une vitesse de 25 mm/min est appliquée jusqu'à rupture de la pièce. L'uniaxial tensile strength est reporté (max. force / surface sectionnelle).

Un T Peel Test est réalisé. Des spécimens de 2 cm × 6 cm sont découpés. L'enduction de P4HB est séparée de l'implant biologique sur une section de 1,5 cm × 2 cm. Les 2 morceaux ainsi séparés sont placés dans les mords pneumatiques (45 psi). La section de l'implant biologique enduite est séparée à une vitesse de 25 mm/min. La T-peel force est mesurée sur une largeur normalisée de 20 mm et sur une moyenne de 5 pics (charges). Sur les 3 spécimens testés, l'adhésion était plus forte de sorte que le test n'a pas permis de peler la couche enduite.

### Exemple 10 : exemple de séchage et pressage et caractéristiques des dispositifs médicaux obtenus

Selon les mêmes conditions de tests précédentes, une matrice dermique traitée selon l'exemple 4 est enduite à approximativement 0,03 g/cm² de P4HB puis pressée pendant 60 s à 200 bar et 100°C. On obtient une valeur UTS max moyenne sur 3 éprouvettes de 27,65 MPa.

### Exemple 11 :

Selon les mêmes conditions de tests précédentes, une matrice dermique traitée selon l'exemple 5 est enduite à approximativement 0,03 g/cm² de P4HB puis pressée pendant 30 s à 100 bars et 100°C. On obtient une valeur UTS max moyenne sur 3 éprouvettes de 33,07 MPa.

### Exemple 12 : exemple de mise en forme de la matrice avant enduction

Une matrice dermique est hydratée pendant 15 min dans l'eau. La matrice est ensuite positionnée sur une forme et maintenue sur ladite forme par un disque ou une contre-forme (figures 1a, 1b et 1c). L'ensemble est lyophilisé pour obtenir un dispositif avec le taux d'humidité résiduel inférieur à 18%. La matrice est ensuite positionnée sur une presse pour pressage sur une forme à 6 bars à une température de 190°C pendant 60s. Sur 3 éprouvettes testées, d'une épaisseur moyenne 1,35 mm, l' « uniaxial tensile strenght » (UTS) max moyen est de 26,50 MPa. Le test est réalisé sur un banc de mesure Instron modèle 3342/L2345. Le spécimen est découpé avec un emporte-pièce type bone shape type V cutter tel que décrit dans le standard ASTM D -638-5. La pièce ainsi découpée est introduite par chaque extrémité dans les mords pneumatiques du banc (60psi) en laissant une partie centrale de 2,5 cm. Une vitesse de 25 mm/min est appliquée jusqu'à rupture de la pièce. L'« uniaxial tensile strength » est reporté (max. force / surface sectionnelle).

Selon un mode de préparation décrit auparavant dans les exemples précédents, la matrice dermique peut ensuite être traitée de façon à permettre l'adhésion du P4HB sur la forme ainsi créée par la méthode décrite également dans les exemples précédents.

## Revendications

1. Procédé de fabrication d'un dispositif médical en trois dimensions à partir d'une matrice biologique acellulaire **caractérisé en ce qu'**il comprend au moins la succession des étapes suivantes :
a/ mouiller une matrice biologique acellulaire avec un liquide, et
b/ conformer la matrice biologique acellulaire mouillée de façon à ce qu'elle prenne une forme en trois dimensions.

2. Procédé de fabrication d'un dispositif médical selon la revendication 1, **caractérisé en ce que** l'étape a/ consistant à mouiller la matrice biologique acellulaire est réalisée en trempant la matrice biologique acellulaire dans un volume d'eau permettant son immersion.

3. Procédé de fabrication d'un dispositif médical selon la précédente revendication, **caractérisé en ce que** le trempage est réalisé pendant une durée 30 secondes à 20 minutes.

4. Procédé de fabrication d'un dispositif médical selon l'une des précédentes revendications, **caractérisé en ce que** l'étape a/ est réalisée avec de l'eau apyrogène.

5. Procédé de fabrication d'un dispositif médical selon l'une des précédentes revendications, **caractérisé en ce que** l'étape a/ est réalisée de façon à obtenir une matrice biologique acellulaire de couleur grise.

6. Procédé de fabrication d'un dispositif médical selon l'une des précédentes revendications, **caractérisé en ce que** l'étape b/ de conformage est réalisée par formage par le vide et/ou moulage humide et/ou pressage.

7. Procédé de fabrication d'un dispositif médical selon l'une des précédentes revendications, **caractérisé en ce que** la matrice biologique acellulaire est d'origine humaine ou animale.

8. Procédé de fabrication d'un dispositif médical selon la précédente revendication, **caractérisé en ce que** la matrice biologique acellulaire d'origine animale est choisie parmi les matrices biologiques acellulaires d'origine porcine, bovine, équine, caprine, de poissons et leurs mélanges.

9. Procédé de fabrication d'un dispositif médical selon l'une des précédentes revendications, **caractérisé en ce que** la matrice biologique acellulaire est choisie parmi l'une des matrices biologiques suivantes : derme, sous-muqueuse intestinale, aorte, vessie, membrane amniotique, péritoine, péricarde, dure-mère, tendons, cartilage et leurs mélanges.

10. Procédé de fabrication d'un dispositif médical selon l'une des précédentes revendications, **caractérisé en ce qu'**il comprend :
- avant ou après l'étape a/ ou avant ou après l'étape b/, une étape de préparation de la matrice biologique acellulaire de façon à permettre l'adhérence sur ladite matrice biologique acellulaire d'une solution comprenant au moins un polymère, et
- avant ou après l'étape a/ ou avant ou après l'étape b/, et après l'étape de préparation de la matrice biologique acellulaire, une étape d'enduction partielle ou totale de la matrice biologique acellulaire avec une solution comprenant au moins un polymère.

11. Procédé de fabrication d'un dispositif médical selon la précédente revendication, **caractérisé en ce que** l'enduction est réalisée à une température inférieure ou égale à la température de dénaturation du collagène.

12. Procédé de fabrication d'un dispositif médical selon la précédente revendication, **caractérisé en ce que** l'enduction est réalisée à une température comprise entre 10 et 60°C.

13. Procédé de fabrication d'un dispositif médical selon l'une des revendications 10 à 12 **caractérisé en ce qu'**avant l'enduction et/ou après l'enduction, la matrice biologique acellulaire est séchée de façon à présenter un taux d'humidité résiduel compris entre 10% et 18%.

14. Procédé de fabrication d'un dispositif médical selon l'une des revendications 10 à 13, **caractérisé en ce que** l'étape de préparation de la matrice biologique cellulaire mouillée de façon à permettre l'adhérence sur ladite matrice biologique acellulaire d'une solution comprenant au moins un polymère, consiste en un traitement de surface chimique et/ou mécanique et/ou électrochimique et/ou physique.

15. Procédé de fabrication d'un dispositif médical selon l'une des revendications 10 à 14, **caractérisé en ce que** l'enduction est réalisée par enduction par coulage, enduction par vaporisation ou enduction par immersion.

16. Procédé de fabrication d'un dispositif médical selon l'une des revendications 10 à 15, **caractérisé en ce que** la solution comprenant au moins un polymère a été préalablement obtenue par solubilisation du ou des polymère(s) dans au moins un solvant polaire.

17. Procédé de fabrication d'un dispositif médical selon la précédente revendication, **caractérisé en ce qu'**après solubilisation du ou des polymère(s), la solution est dégazée et/ou débullée sous vide pour purger le mélange de bulles d'air.

18. Procédé de fabrication d'un dispositif médical selon l'une des revendications 10 à 17, caractérisé en ce la solution de polymère(s) comprend un ou plusieurs polymères choisis parmi les polymères suivants : polyhydroxyalkanoates, poly(glycolides), poly(lactide-co-glycolides), poly(lactic acid), poly(glycolic acid), poly(lactic acid-co-glycolic acids), polycaprolactones, poly(orthoesters), polyanhydrides, poly(phosphazenes), polyhydroxyalkanoates, polyesters, poly(lactide-co-caprolactones), polycarbonates; tyrosine polycarbonates, polyamides, polyesteramides, poly(dioxanones), poly(alkylene alkylates), polyethers, polyvinyl pyrrolidones, polyurethanes, polyetheresters, polyacetals, polycyanoacrylates, poly(oxyethylene), copolymères de poly(oxypropylene), polyacetals, polyketals, polyphosphates, polyphosphoesters, polyalkylene oxalates, polyalkylene succinates, poly(maleic acids), chitin, chitosan, et leurs mélanges.

19. Procédé de fabrication d'un dispositif médical selon l'une des revendications 10 à 18, caractérisé en ce la solution de polymère(s) comprend au moins du P4HB solubilisé dans une solution d'acétone.

20. Dispositif médical en trois dimensions obtenu par la mise en oeuvre du procédé selon l'une des revendications 1 à 19, **caractérisé en ce qu'**il comprend au moins une matrice biologique acellulaire conformée en trois dimensions.

21. Dispositif médical en trois dimensions selon la précédente revendication, **caractérisé en ce qu'**il ne comprend pas de support pour le maintien en trois dimensions de la matrice biologique.

22. Dispositif médical en trois dimensions selon la revendication 20 ou 21, **caractérisé en ce que** la matrice biologique acellulaire conformée en trois dimensions est recouverte partiellement ou totalement par au moins une couche comprenant au moins un polymère.

23. Dispositif médical en trois dimensions selon l'une des revendications 20 à 22, **caractérisé en ce qu'**il s'agit d'un implant pour reconstruction mammaire.

## Patentansprüche

1. Verfahren zum Herstellen einer dreidimensionalen medizinischen Vorrichtung aus einer azellulären biologischen Matrix,
**dadurch gekennzeichnet, dass** es mindestens die Abfolge der folgenden Schritte umfasst:
a/ Benetzen einer azellulären biologischen Matrix mit einer Flüssigkeit und
b/ Formen der benetzten azellulären biologischen Matrix derart, dass sie eine dreidimensionale Form annimmt.

2. Verfahren zum Herstellen einer medizinischen Vorrichtung nach Anspruch 1, **dadurch gekennzeichnet, dass** der Schritt a/, der darin besteht, die azelluläre biologische Matrix zu befeuchten, durch Eintauchen der azellulären biologischen Matrix in ein Wasservolumen, das ihr vollständige Eintauchen gestattet, erfolgt.

3. Verfahren zum Herstellen einer medizinischen Vorrichtung nach dem vorhergehenden Anspruch, **dadurch gekennzeichnet, dass** das Eintauchen über eine Dauer von 30 Sekunden bis 20 Minuten erfolgt.

4. Verfahren zum Herstellen einer medizinischen Vorrichtung nach einem der vorhergehenden Ansprüche, **dadurch gekennzeichnet, dass** der Schritt a/ mit pyrogenfreiem Wasser erfolgt.

5. Verfahren zum Herstellen einer medizinischen Vorrichtung nach einem der vorhergehenden Ansprüche, **dadurch gekennzeichnet, dass** der Schritt a/ derart erfolgt, dass eine azelluläre biologische Matrix grauer Farbe erhalten wird.

6. Verfahren zum Herstellen einer medizinischen Vorrichtung nach einem der vorhergehenden Ansprüche, **dadurch gekennzeichnet, dass** der Formgebungsschritt b/ durch Vakuumformen und/oder Nassformen und/oder Pressen erfolgt.

7. Verfahren zum Herstellen einer medizinischen Vorrichtung nach einem der vorhergehenden Ansprüche, **dadurch gekennzeichnet, dass** die azelluläre biologische Matrix menschlichen oder tierischen Ursprungs ist.

8. Verfahren zum Herstellen einer medizinischen Vorrichtung nach dem vorhergehenden Anspruch, **dadurch gekennzeichnet, dass** die azelluläre biologische Matrix tierischen Ursprungs aus azellulären biologischen Matrizen, die vom Schwein, Rind, Pferd, der Ziege, Fischen und deren Mischungen stammen, ausgewählt wird.

9. Verfahren zum Herstellen einer medizinischen Vorrichtung nach einem der vorhergehenden Ansprüche, **dadurch gekennzeichnet, dass** die azelluläre biologische Matrix aus einer der folgenden biologischen Matrizen ausgewählt wird: Dermis, intestinale Submukosa, Aorta, Blase, Amnionmembran, Peritoneum, Perikard, Dura mater, Sehnen, Knorpel und deren Mischungen.

10. Verfahren zum Herstellen einer medizinischen Vorrichtung nach einem der vorhergehenden Ansprüche, **dadurch gekennzeichnet, dass** es Folgendes umfasst:
- vor oder nach Schritt a/ oder vor oder nach Schritt b/, einen Schritt des Herstellens der azellulären biologischen Matrix derart, dass eine Adhäsion einer mindestens ein Polymer umfassenden Lösung an der azellulären biologischen Matrix erfolgen kann, und
- vor oder nach Schritt a/ oder vor oder nach Schritt b/ und nach dem Schritt des Herstellens der azellulären biologischen Matrix, einen Schritt des teilweisen oder vollständigen Beschichtens der azellulären biologischen Matrix mit einer mindestens ein Polymer umfassenden Lösung.

11. Verfahren zum Herstellen einer medizinischen Vorrichtung nach dem vorhergehenden Anspruch, **dadurch gekennzeichnet, dass** das Beschichten bei einer Temperatur unter oder gleich der Denaturierungstemperatur des Kollagens erfolgt.

12. Verfahren zum Herstellen einer medizinischen Vorrichtung nach dem vorhergehenden Anspruch, **dadurch gekennzeichnet, dass** das Beschichten bei einer Temperatur zwischen 10 und 60 °C erfolgt.

13. Verfahren zum Herstellen einer medizinischen Vorrichtung nach einem der Ansprüche 10 bis 12 , **dadurch gekennzeichnet, dass** die azelluläre biologische Matrix vor dem Beschichten und/oder nach dem Beschichten so getrocknet wird, dass sie eine Restfeuchtigkeit zwischen 10 % und 18 % aufweist.

14. Verfahren zum Herstellen einer medizinischen Vorrichtung nach einem der Ansprüche 10 bis 13, **dadurch gekennzeichnet, dass** der Schritt des Herstellens der benetzten zellulären biologischen Matrix derart, dass eine Adhäsion einer mindestens ein Polymer umfassenden Lösung an der azellulären biologischen Matrix erfolgen kann, in einer chemischen und/oder mechanischen und/oder elektrochemischen und/oder physikalischen Oberflächenbehandlung besteht.

15. Verfahren zum Herstellen einer medizinischen Vorrichtung nach einem der Ansprüche 10 bis 14, **dadurch gekennzeichnet, dass** das Beschichten durch Gießbeschichtung, Aufdampfbeschichtung oder Tauchbeschichtung erfolgt.

16. Verfahren zum Herstellen einer medizinischen Vorrichtung nach einem der Ansprüche 10 bis 15, **dadurch gekennzeichnet, dass** die mindestens ein Polymer umfassende Lösung zuvor durch Solubilisieren des/der Polymer(e) in mindestens einem polaren Lösungsmittel erhalten wurde.

17. Verfahren zum Herstellen einer medizinischen Vorrichtung nach dem vorhergehenden Anspruch, **dadurch gekennzeichnet, dass** nach dem Solubilisieren des/der Polymer(e) die Lösung unter Vakuum entgast und/oder von Blasen befreit wird, um Luftblasen aus dem Gemisch zu entfernen.

18. Verfahren zum Herstellen einer medizinischen Vorrichtung nach einem der Ansprüche 10 bis 17, **dadurch gekennzeichnet, dass** die Lösung von Polymer(en) ein oder mehrere Polymere umfasst, die aus den folgenden Polymeren ausgewählt werden: Polyhydroxyalkanoate, Poly(glycolide), Poly(lactid-co-glykolide), Poly(milchsäure), Poly(glykolsäure), Poly(lactid-co-glycolid), Polycaprolactone, Poly(orthoester), Polyanhydride, Poly(phosphazene), Polyhydroxyalkanoate, Polyester, Poly(lactid-co-caprolactone), Polycarbonate; Tyrosinpolycarbonate, Polyamide, Polyesteramide, Poly(dioxanone), Poly(alkylenalkylate), Polyether, Polyvinylpyrrolidone, Polyurethane, Polyetherester, Polyacetale, Polycyanoacrylate, Poly(oxyethylen), Poly(oxypropylen)-Copolymere, Polyacetale, Polyketale, Polyphosphate, Polyphosphoester, Polyalkylenoxalate, Polyalkylensuccinate, Poly(maleinsäuren), Chitin, Chitosan und Mischungen davon.

19. Verfahren zum Herstellen einer medizinischen Vorrichtung nach einem der Ansprüche 10 bis 18, **dadurch gekennzeichnet, dass** die Lösung des/der Polymer(e) mindestens in einer Acetonlösung gelöstes P4HB enthält.

20. Dreidimensionale medizinische Vorrichtung, die durch Anwendung des Verfahrens nach einem der Ansprüche 1 bis 19 erhalten wird,
**dadurch gekennzeichnet, dass** sie mindestens eine dreidimensional geformte azelluläre biologische Matrix umfasst.

21. Dreidimensionale medizinische Vorrichtung nach dem vorhergehenden Anspruch, **dadurch gekennzeichnet, dass** sie keinen Träger umfasst, um die biologische Matrix in drei Dimensionen zu halten.

22. Dreidimensionale medizinische Vorrichtung nach Anspruch 20 oder 21, **dadurch gekennzeichnet, dass** die dreidimensional geformte azelluläre biologische Matrix teilweise oder vollständig von mindestens einer mindestens ein Polymer umfassenden Schicht bedeckt ist.

23. Dreidimensionale medizinische Vorrichtung nach einem der Ansprüche 20 bis 22, **dadurch gekennzeichnet, dass** sie ein Implantat zur Brustrekonstruktion ist.

## Claims

1. A method for manufacturing a three-dimensional medical device from an acellular biological matrix **characterized in that** it comprises at least the following sequence of steps:
a/ wetting an acellular biological matrix with a liquid, and
b/ shaping the wet acellular biological matrix so that it takes on a three-dimensional shape.

2. The method for manufacturing a medical device according to claim 1, **characterized in that** step a/ consisting in wetting the acellular biological matrix is carried out by soaking the acellular biological matrix in a volume of water allowing it to be immersed.

3. The method for manufacturing a medical device according to the preceding claim, **characterized in that** the soaking is carried out for a period of 30 seconds to 20 minutes.

4. The method for manufacturing a medical device according to one of the preceding claims, **characterized in that** step a/ is carried out with pyrogen-free water.

5. The method for manufacturing a medical device according to one of the preceding claims, **characterized in that** step a/ is carried out so as to obtain an acellular biological matrix gray in color.

6. The method for manufacturing a medical device according to one of the preceding claims, **characterized in that** step b/ of shaping is carried out by vacuum forming and/or wet molding and/or pressing.

7. The method for manufacturing a medical device according to one of the preceding claims, **characterized in that** the acellular biological matrix is of human and/or animal origin.

8. The method for manufacturing a medical device according to the preceding claim, **characterized in that** the acellular biological matrix of animal origin is selected from among the acellular biological matrices of porcine, bovine, equine, caprine, or fish origin, and mixtures thereof.

9. The method for manufacturing a medical device according to one of the preceding claims, **characterized in that** the acellular biological matrix is selected from one of the following biological matrices: dermis, intestinal submucosa, aorta, bladder, amniotic membrane, peritoneum, pericardium, dura mater, tendons, cartilage, and mixtures thereof.

10. The method for manufacturing a medical device according to one of the preceding claims, **characterized in that** it comprises:
- before or after step a/ or before or after step b/, a step for preparing the acellular biological matrix so as to allow adhesion to said acellular biological matrix of a solution comprising at least one polymer, and
- before or after step a/ or before or after step b/, and after the step of preparing the acellular biological matrix, a step of coating the acellular biological matrix in full or in part with a solution comprising at least one polymer.

11. The method for manufacturing a medical device according to the preceding claim, **characterized in that** the coating is performed at a temperature lower than or equal to the denaturation temperature of the collagen.

12. The method for manufacturing a medical device according to the preceding claim, **characterized in that** the coating is performed at a temperature between 10 and 60°C.

13. The method for manufacturing a medical device according to one of claims 10 to 12, **characterized in that** before coating and/or after coating, the acellular biological matrix is dried so as to have a residual moisture content of between 10% and 18%.

14. The method for manufacturing a medical device according to one of claims 10 to 13, **characterized in that** the step of preparing the wet cellular biological matrix so as to allow adhesion to said acellular biological matrix of a solution comprising at least one polymer, consists of a chemical and/or mechanical and/or electrochemical and/or physical surface treatment.

15. The method for manufacturing a medical device according to one of claims 10 to 14, **characterized in that** the coating is carried out by solvent casting, spray coating, or dip coating.

16. The method for manufacturing a medical device according to one of claims 10 to 15, **characterized in that** the solution comprising at least one polymer was obtained beforehand through solubilization of the polymer(s) in at least one polar solvent.

17. The method for manufacturing a medical device according to the preceding claim, **characterized in that,** after solubilization of the polymer(s), the solution is degassed and/or debubbled under vacuum in order to purge the mixture of air bubbles.

18. The method for manufacturing a medical device according to one of claims 10 to 17, **characterized in that** the solution of polymer(s) comprise one or more polymers selected from among the following polymers: polyhydroxyalkanoates, poly(glycolides), poly(lactide-co-glycolides), poly(lactic acid), poly(glycolic acid), poly(lactic acid-co-glycolic acids), polycaprolactones, poly(orthoesters), polyanhydrides, poly(phosphazenes), polyhydroxyalkanoates, polyesters, poly(lactide-co-caprolactones), polycarbonates; tyrosine polycarbonates, polyamides, polyesteramides, poly(dioxanones), poly(alkylene alkylates), polyethers, polyvinyl pyrrolidones, polyurethanes, polyether esters, polyacetals, polycyanoacrylates, poly(oxyethylene), copolymers of poly(oxypropylene), polyacetals, polyketals, polyphosphates, polyphosphoesters, polyalkylene oxalates, polyalkylene succinates, poly(maleic acids), chitin, chitosan, and mixtures thereof.

19. The method for manufacturing a medical device according to one of claims 10 to 18, **characterized in that** the polymer solution comprises at least P4HB dissolved in an acetone solution.

20. A three-dimensional medical device, obtained by implementing the method according to one of claims 1 to 19, **characterized in that** it comprises at least one three-dimensionally shaped acellular biological matrix.

21. The three-dimensional medical device according to the preceding claim, **characterized in that** it does not comprise a support for maintaining the biological matrix in three dimensions.

22. The three-dimensional medical device according to claim 20 or 21, **characterized in that** the three-dimensionally shaped acellular biological matrix is covered in full or in part by at least one layer comprising at least one polymer.

23. The three-dimensional medical device according to one of claims 20 to 22, **characterized in that** it is an implant for breast reconstruction.
